# EUROPEAN PATENT SPECIFICATION

(11) **EP 1 534 253 B1**
(45) Date of publication and mention of the grant of the patent: **28.05.2008**
(21) Application number: 03766550.2
(22) Date of filing: 16.07.2003
(51) Int. Cl.: A61K 9/70

(54) **PROCESS FOR MAKING ORALLY CONSUMABLE DOSAGE FORMS**
VERFAHREN ZUR HERSTELLUNG VON ORAL VERZEHRBAREN DARREICHUNGSFORMEN
PROCEDE POUR REALISER DES DOSES POSOLOGIQUES S'ADMINISTRANT PAR VOIE ORALE

(30) Priority: 26.07.2002 GB 0217382
(43) Date of publication of application: 01.06.2005
(73) Proprietor: Pfizer Limited, Sandwich, Kent CT13 9NJ (GB); PFIZER INC., New York, N.Y. 10017 (US)
(72) Inventor: AUFFRET, Anthony, David, Sandwich, Kent, CT13 9NJ (GB); BENEE, Lisa, Suzanne, Sandwich, Kent CT13 9NJ (GB)
(74) Representative: Wood, David John
(86) International application number: PCT/IB2003/003244
(87) International publication number: WO 2004/012720

(56) References cited:
- EP-A- 0 256 611
- WO-A-00/18365
- WO-A-01/70194
- WO-A-02/43657
- WO-A-03/030881
- US-A- 5 518 902
- US-A1- 2003 107 149

## Description

The present invention is concerned with a process for making rapidly dissolving and dispersing dosage forms, particularly orally consumable films, for the delivery of pharmaceutically active agents and with the dosage forms so obtained.

The use of orally consumable dosage forms, particularly films, to deliver pharmaceutically active agents is well known in the art.

Thus WO 98/20862 describes a preparation for application in the oral cavity with one layer or film which adheres to the mucous membrane, characterised in that the adhesive layer or film contains a homogenous mixture consisting of a water soluble polymer, a mixture of non-ionic surface active materials, a polyalcohol, a cosmetic or pharmaceutical active substance, and a food flavouring or aromatic agent.

WO 98/26780 describes a solid medicament preparation which can decompose in aqueous media and has a flat-, foil-, paper- or wafer-type presentation for the application and release of active substances in the buccal cavity. The invention is characterised in that it contains buprenorphine, or an active substance which is pharmacologically comparable thereto, or a therapeutically suitable salt of buprenorphine or of the pharmacologically comparable active substance.

WO 98/26763 describes a medicament preparation with a flat-, paper- or wafer-like presentation for the application and release of active substances into the buccal cavity. The preparation is characterised in that it contains apomorphine or one of its therapeutically suitable salts.

WO 99/17753 describes a rapidly soluble filmy preparation comprising a drug, an edible and readily soluble high-molecular substance and a sugar which is rapidly soluble in the oral cavity.

WO 00/18365 describes physiologically acceptable films, including edible films, which include a water-soluble film-forming polymer such as pullulan. Edible films including pullulan and antimicrobially effective amounts of the essential oils thymol, methyl salicylate, eucalyptol and menthol are effective at killing the plaque-producing germs that cause dental plaque, gingivitis and bad breath. The film can also contain pharmaceutically active agents.

WO 01/70194 describes physiologically acceptable films, including edible films, which include a water-soluble film-forming polymer, such as pullulan, and a taste-masked pharmaceutically active agent, such as dextromethorphan. The taste-masking agent is preferably a sulphonated polymer ion exchange resin comprising polystyrene cross-linked with divinylbenzene, such as Amberlite™.

WO 01/70194 describes a method for preparing the orally consumable film of the invention which comprises
(a) dissolving water-soluble ingredients in water to provide an aqueous solution;
(b) mixing at least one water-soluble film former and at least one stabilising agent to provide a film-forming mixture;
(c) combining the film-forming mixture and the aqueous solution to provide a hydrated polymer gel;
(d) mixing oils to form an oil mixture;
(e) adding the oil mixture to the hydrated polymer gel and mixing to provide a uniform gel;
(f) casting the uniform gel on a substrate; and
(g) drying the cast to provide a film.

The difficulty associated with a process of this type is that a high viscosity composition, typically a gel, is required in order to achieve a satisfactory cast. It follows that the resulting dosage form gives rise to a viscous solution when placed in the mouth of the consumer. This may be satisfactory for the delivery of oral healthcare products, such as mouthwashes, which are intended to remain in the mouth for some time, but such dosage forms do not lend themselves to the delivery of pharmaceutically active agents which need to be rapidly dissolved and dispersed as soon as the dosage form is placed in the mouth. In other words, the high viscosity necessary for casting militates against the preparation of dosage forms which rapidly dissolve and disperse in the mouth.

We have now found that by an appropriate choice of film-forming components, specifically pullulan and sodium alginate, it is possible to provide a composition having the viscosity necessary for casting which, by appropriate treatment after casting, gives a dosage form capable of providing a low viscosity solution when placed in the mouth of the consumer. Thus, for the first time, there is provided a process for preparing orally consumable dosage forms which have the handling properties necessary for manufacture and rapidly dissolve and disperse in the mouth.

The dosage forms obtained by the process of the invention may be used for the administration of pharmaceutically active agents to both humans and animals. Of the latter, companion animals, particularly cats, dogs and horses, are considered especially suitable for the administration of drugs in this way.

While primarily intended for the administration of drugs suitable for oral delivery, the dosage forms of the invention may be used for the administration of pharmaceutically active agent(s) to any suitable mucosal surface, for example, the eyes, as well as to wound surfaces.

For the purposes of the present invention, the term 'pharmaceutically active agent' is used to describe any drug which is suitable for the treatment of a human or an animal and includes oral healthcare actives such as deodorising agents, anti-microbial agents and salivary stimulants.

The term 'casting' is used herein to describe the means by which the compositions of the invention are shaped into dosage forms. Typically, the composition of the invention is cast on a suitable substrate, typically a glass plate, but alternative means such as extrusion through a slit orifice onto a substrate or the use of a mould may be employed. The requirements of the invention regarding viscosity are the same regardless of the means by which the compositions are cast.

Viscosity (Pa.s) may be defined as the shear stress (Pa) of a solution or composition divided by the shear rate (s⁻¹) at which the shear stress is measured.

For the purposes of this invention, the terms 'high' and 'low' viscosity are defined in terms of the difference in shear stress between the composition used for casting and the solution formed in the mouth. The term 'low' is employed when the viscosity of the solution formed in the mouth is less than 80% that of the composition used for casting, both being measured at a shear rate of 100s⁻¹ and, in respect of measurement of the casting composition, after the composition has been allowed to stand for 24 hours. [It is not a necessary feature of the invention that the casting composition be allowed to stand for this period, but it serves as a convenient point in time at which to measure viscosity.]

According to the present invention, therefore, there is provided a process for preparing an orally consumable dosage form which affords a low viscosity solution when placed in the mouth of the consumer, which process comprises the steps of
(a) preparing a hydrated polymer composition comprising pullulan and sodium alginate having a viscosity suitable for casting;
(b) casting said composition into the shape of a dosage form; and
(c) drying said dosage form under such conditions as to provide a dosage form which rapidly dissolves and disperses in the mouth of the consumer.

In the manufacture of soft centre chocolates, a thick paste containing an enzyme is used to form the centre of the confection. In the period between manufacture and consumption, the enzyme degrades the substance of the paste to give a liquid centre. Such technology may be used to provide a dosage form which affords a low viscosity solution when placed In the mouth of the consumer.

Thus according to preferred embodiment of the Invention, there is provided a process which comprises the steps of
(a) preparing a hydrated polymer composition comprising pullulan, sodium alginate and one or more pharmaceutically active agents, which composition additionally comprises one or both of the enzymes pullulanase and alginate lyase;
(b) casting said composition while still viscous into the shape of a dosage form; and
(c) drying said dosage form to provide a form which rapidly dissolves and disperses in the mouth of the consumer.

Some materials are known to be unstable to radiation. Sodium alginate: formulations, for example, reduce in viscosity when exposed to gamma-radiation. Thus a viscous mixture of pullulan and sodium alginate may be used for casting and the viscosity of the alginate component subsequently reduced by gamma-irradiation, typically 25 kGy or 40kGy, to give a dosage form which affords a low viscosity solution when placed in the mouth of the consumer.

Thus according to a second preferred embodiment, there is provided a process which comprises the steps of
(a) preparing a hydrated polymer composition comprising pullulan, sodium alginate and one or more pharmaceutically active agents;
(b) casting said composition into the shape of a dosage form;
(c) drying said dosage form; and
(d) irradiating said dosage form with gamma-radiation to provide a form which rapidly dissolves and disperses in the mouth of the consumer.

Also within the scope of the present invention are dosage forms, particularly orally consumable films, prepared by the process of the invention. The dosage forms of the invention dissolve in the mouth to form a low viscosity solution which rapidly disperses the pharmaceutically active agent(s) contained therein.

Protection is especially sought for orally consumable dosage forms according to the invention which contain Ibuprofen, ivermectin, or any form of eletriptan (including the free base, salts and polymorphs thereof).

Dosage forms, particularly orally-consumable films, wherein the pharmaceutically active agent is the anti-migraine drug eletriptan hydrobromide (Relpax™) or eletriptan hemisulphate are especially preferred.

As indicated, the process of the invention requires that the composition used for manufacture of the dosage form has a higher viscosity than the solution produced In the mouth of the consumer.

The dosage forms of the invention typically comprise the film-forming agents pullulan and sodium alginate, one or more pharmaceutically active agents and at least one of the following additional agents: plasticising agent, saliva-stimulating agent, cooling agent, surfactant, emulsifying agent, sweetener, flavouring and/or fragrance, colouring agent, preservative, a triglyceride, a polyethylene oxide and propylene glycol.

Pullulan is a bioadhesive polysaccharide commonly employed in the preparation of orally consumable dosage forms and is used in the dosage forms of the invention in an amount of up to 70 wt%, preferably from 5 to 45 wt%, more preferably from 15 to 25 wt% and most preferably about 20 wt%.

Sodium alginate is a naturally-occurring copolymer of mannuronic and guluronic acid salts. It is water-soluble above pH 4.0, but under more acidic conditions is converted to the insoluble, but water-swellable, alginic acid. It is used in the dosage forms of the invention in an amount of up to 5.0 wt%, preferably from 0.1 to 2.5 wt% and most preferably about 0.5 wt%.

Pharmaceutically active agents which may be delivered using dosage forms prepared by the process of the invention include
analgesic anti-pyretics;
anti-diarrhoeals;
anti-histamines;
anti-microbials;
anti-Parkinsonism drugs;
anti-tussives/cough suppressants;
bronchodilators;
decongestants;
drugs which selectively modify CNS function;
drugs for treating gastric disorders;
expectorants;
general non-selective CNS depressants;
general non-selective CNS stimulants;
H₂-antagonists;
narcotic analgesics;
non-steroidal anti-inflammatory drugs;
oral insulin;
proton pump inhibitors;
psychopharmacological drugs; and
wound-healing drugs

Specific examples of the foregoing drugs are to be found in the aforementioned WO 01/70194.

Other actives which may be delivered using dosage forms prepared by the process of the invention include
Anti-cholesterolaemics, for example, Lipitor™;
anti-emetics, for example, ondansetron;
anti-fungals, for example, fosfluconazole;
anti-infectives other than anti-microbial agents, for example, azithromycin;
anti-inflammatories, for example, Rimidil™;
anti-parasitic agents, for example, Pyrantel™;
anti-pyretics other than analgesic anti-pyretics;
appetite stimulants, for example, megatrol acetate;
cardiovascular drugs (including anti-hypertensives), for example, Norvasc™;
drugs for renal failure, for example, frusemide; and
PDE5 inhibitors, for example, Viagra™.

The dosage forms of the invention may contain one or more pharmaceutically active agents, which agents may or may not be of the same therapeutic type. Thus a dosage form according to the invention could contain an anti-tussive plus an anti-histamine, a nasal decongestant or bronchodilator, an analgesic, an anti-inflammatory, a cough suppressant and/or an expectorant.

The amount of pharmaceutically active agent provided in each dosage form will obviously be dependent on the dose needed to provide an effective amount. Furthermore, the amount provided may be adjusted to deliver a predetermined dose over a predetermined period of time. The concentration of active agent(s) for pharmaceutical and veterinary products in accordance with the invention may be up to 75% w/w, but is typically in the range 0.1 to 50% w/w. Typical doses which can be delivered per dosage form are in the range 10µg to 100mg.

The dosage forms of the invention may also be used to deliver oral healthcare products, such a deodorising agents, anti-microbial agents and salivary stimulants. The concentration of active agent(s) for oral healthcare products is typically in the range 0.1 to 15% w/w. Typical doses which can be delivered per dosage form are comparable to those for pharmaceutical and veterinary products, viz. from 10µg to 100mg.

Preferred pharmaceutically active agents for delivery by means of the dosage forms of the invention include ibuprofen, ivermectin and any form of eletriptan (including the free base, salts and polymorphs thereof).

The anti-migraine drugs eletriptan hydrobromide (Relpax™) and eletriptan hemisulphate are especially preferred for delivery by this means. Thus a dosage form, typically an orally consumable film, prepared according to the process of the invention may be used to deliver an effective amount of Relpax™ to a migraine sufferer in need of such treatment. For a film prepared by the process of the invention measuring 2.2cm x 3.2cm and weighing from 60 to 190mg, the typical adult dose of Relpax™ would be in the range 5 to 80mg.

Preferred plasticising agents include monoacetin, diacetin and triacetin, polyalcohols, such as glycerol and glycerol monoesters, and sorbitol, which may be present in the dosage forms of the invention in an amount of from 0 to 20 wt%, preferably from 0 to 2 wt%.

Preferred saliva-stimulating agents include citric, lactic, malic, succinic, ascorbic, adipic, fumaric and tartaric acids which may be present in the dosage forms of the invention in an amount of from 0.01 to 12 wt%, preferably from 1 to 10 wt% and most preferably from 2.5 to 6 wt%.

Preferred cooling agents include monomenthyl succinate, WS3, WS23 and Ultracool II which may be present in the dosage forms of the invention in an amount of from 0.001 to 2.0 wt%, preferably from 0.2 to 0.4 wt%.

Preferred surfactants include mono- and diglycerides of fatty acids, polyoxyethylene sorbitol esters and di- and tri-block copolymers, such as pluronics, which may be present in the dosage forms of the invention in an amount of from 0.5 to 15 wt%, preferably 1 to 5 wt%.

Preferred emulsifying agents include triethanolamine stearate and quaternary ammonium compounds which may be present in the dosage forms of the invention in an amount of from 0 to 5 wt%, preferably from 0.01 to 0.7 wt%.

Suitable sweeteners, both natural and artificial, may be used in the dosage forms of the invention in an amount effective to provide the desired level of sweetness. This amount will typically be in the range 0.01 to 10 wt%, preferably from 2 to 5 wt%.

Suitable flavourings and/or fragrances include those well known in the art, both natural and artificial, which may be used in the dosage forms of the invention in an amount sufficient to give the desired flavour/fragrance. This amount will typically be in the range 0.1 to 30 wt%, preferably from 2 to 25 wt%, most preferably from 8 to 10 wt%.

Suitable colouring agents include those well known in the art, for example, titanium oxide, which may be used in the dosage forms of the invention in an amount sufficient to give the desired colouring. This amount will typically be in the range up to about 5 wt%, preferably less than 1 wt%.

Preferred preservatives include sodium benzoate and potassium sorbate which may be present in the dosage forms of the invention in an amount of from 0.001 to 5 wt%, preferably from 0.01 to 1 wt%.

The dosage forms of the invention may also include a triglyceride, such as olive oil, which may be present in an amount of from 0.1 to 12 wt%, preferably from 0.5 to 9 wt%. They may also contain a polyethylene oxide such as N-10 (Union Carbide) having a molecular weight of from 50,000 to 6,000,000 which may be present in an amount of from 0.1 to 5 wt%, preferably from 0.2 to 4.0 wt%.

The dosage forms of the invention may also contain propylene glycol in an amount of from 1 to 20 wt%, preferably from 5 to 15 wt%.

Finally, it may be desirable to taste-mask the dosage forms of the invention using means well known to those skilled in the art, for example, as described

in A Nanda et al, Indian Journal of Pharmaceutical Sciences, 64(1), 10-17 (2002) and the aforementioned WO 01/70194.

According to the first preferred embodiment, step (a), preparation of the hydrated polymer composition for casting, is typically carried out by
(i) mixing the film-forming ingredients In water and allowing to hydrate;
(ii) dissolving the water-soluble ingredients, including the pullulanase and alginate lyase, in water and adding the aqueous solution to the composition resulting from step (i);
(iii) mixing in the organic ingredients and surfactants.

Alternatively, the pullulanase and alginate lyase may be added, not in step (ii), but in a separate step (iv).

According to the second preferred embodiment, step (a), preparation of the hydrated polymer composition for casting, is typically carried out by
(i) mixing the film-forming Ingredients in water and allowing to hydrate;
(ii) dissolving the water-soluble ingredients in water and adding the aqueous solution to the composition resulting from step (i);
(ill) mixing in the organic ingredients and surfactants.

For the purposes of step (I), the film-forming Ingredients, typically comprising pullulan and sodium alginate, are mixed In water, preferably deionised and at a temperature of from 10°C to 90°C. and allowed to hydrate for from 30 minutes to 48 hours to form a gel. The resulting gel contains from 40 to 80 wt% of water and is cooled to 20-30°C over a period of from 1 to 48 hours.

For the purposes of step (ii), the water-soluble ingredients, typically comprising the pharmaceutically active agent, colouring agent, preservative and sweetener, are dissolved In deionised water at a temperature of from 25°C to 45°C. The amount of water used is typically from 5 to 80 wt% of the final composition.

It is also within the scope of the invention that a pharmaceutically active agent which is of limited solubility in water may be used in step (ii) as a suspension thereof.

Steps (i) and (il) may be transposed, that is, the water-soluble ingredients may be dissolved in water to which the film-fomning Ingredients are added.

For the purposes of step (iii), the organic ingredients and surfactants are typically added in undiluted form to the composition resulting from the preceding step.

The mixture resulting from steps (i) to (iii) is then emulsified by vigorous stirring and, for the purpose of making a dosage form in accordance with the invention, cast, typically within 24 hours of gel preparation, on a suitable substrate, typically a glass plate, preferably covered with an appropriate backing paper.

The resulting film is then dried, typically within 24 hours of casting, in a fan oven at a temperature of from 50°C to 80°C, preferably about 60°C, for from 15 to 90 minutes, or in a coating machine, such as a Labcoater Type LTE-S manufactured by Wemer Mathis AG Oberhasli Switzerland or similar, at a temperature of from 20°C to 150°C, cut to the desired dimensions, packaged and stored. The final film ideally contains from 0.1 to 10 wt% moisture, preferably from 3 to 8 wt% and most preferably from 4 to 7 wt%.

The Invention is illustrated by reference to the following Examples which are not intended to be limiting in any way.

### EXAMPLE 1

A 16.4 wt% pullula/1.64 wt% sodium alginate composition was prepared as follows:

Pullulan (20.0g) and sodium alginate (2.0g) were added to deionised water (100ml) and the mixture left to equilibrate overnight. Pullulanase (125µL, 400 units/mL) and alginate lyase (0.5mg) were added to the resulting gel and the mixture stirred vigorously. During this procedure, a pharmaceutically active agent may be added to the mixture.

The gel remained sufficiently viscous for the purposes of casting for a period of 10-15 minutes, though by judicious selection of enzyme concentrations this period could be extended from, say, 60 minutes to several hours should the casting time require it.

A film in accordance with the invention was prepared by applying the gel to a glass plate, coated with an appropriate backing paper, using a CAMAG hand-operated coater having a 0.5mm gate. The resulting film was dried in a fan oven at 65°C for 25 minutes.

When placed In the mouth, the rehydrated film gave a low viscosity solution which rapidly dissolved and dispersed.

### EXAMPLE 2

A 16.4 wt% pullulan/1.64 wt% sodium alginate composition was prepared as follows: .

Pullulan (20.0g) and sodium alginate (2.0g) were added to deionised water (100mL) and the mixture left to equilibrate overnight. During this procedure, a pharmaceutical active agent may be added to the mixture.

A film in accordance with the invention was prepared by applying the gel to a glass plate, coated with an appropriate backing paper, using a CAMAG hand-operated coater having a 0.5mm gate. The resulting film was dried in a fan oven at 65°C for 25 minutes and then gamma-irradiated at 25 kGy or 40 kGy.

When placed In the mouth, the rehydrated film gave a low viscosity solution which rapidly dissolved and dispersed.

## Claims

1. A process for preparing a dosage form which affords a low viscosity solution when placed in the mouth of the consumer, which process comprises the steps of
(a) preparing a hydrated polymer composition comprising up to 70 wt% pullulan, up to 6.0 wt% sodium alginate and one or more pharmaceutically active agents, which composition additionally comprises one or both of the enzymes pullulanase or alginate lyase;
(b) casting said composition into the shape of a dosage form; and
(c) drying said dosage form to provide a form which rapidly dissolves and disperses in the mouth of the consumer.

2. A process for preparing a dosage form which affords a low viscosity solution when placed In the mouth of the consumer, which process comprises the steps of
(a) preparing a hydrated polymer composition comprising up to 70 wt% pullulan, up to 5.0 wt% sodium alginate and one or more pharmaceutically active agents;
(b) casting said composition into the shape of a dosage form;
(c) drying said dosage form: and
(d) irradiating said dosage form with gamma-radiation to provide a form which rapidly dissolves and disperses in the mouth of the consumer.

3. A process according to Claim 2, wherein said gamma-irradiation is h an amount of 25 kGy or 40 kGy.

4. A process according to any of Claims 1 to 3, wherein the solution formed upon dissolution of the resulting dosage form in the mouth of the consumer has a viscosity which is less than 80% that of the composition formed in step (a).

5. A process according to any of Claims 1 to 4, wherein step (c) is carried out in a fan oven at a temperature of mom 50°C to 80°C for a period of from 15 to 90 minutes.

6. A process according to any of Claims 1 to 4, wherein step (c) is carried out in a coating machine at a temperature of from 20°C to 150°C.

7. A dosage form obtainable according to a process described In any of Claims 1 to 6.

8. A dosage form according to Claim 7, wherein pullulan is present in an amount of from 5 to 45 wt%.

9. A dosage form according to Claim 8, wherein pullulan is present in an amount of from 15 to 25 wt%.

10. A dosage form according to Claim 9, wherein pullulan is present In an amount of 20 wt%.

11. A dosage form according to Claim 7, wherein sodium alginate is present In an amount of from 0.1 to 2.5 wt%.

12. A dosage form according to Claim 11, wherein sodium alginate is present in an amount of 0.5 wt%.

13. A dosage form according to any of Claims 7 to 12, wherein the pharmaceutically active agent is
an anti-cholesterolaemic;
an anti-diarrhoeal;
an anti-ermetic;
an anti-fungal;
an anti-histamine;
an anti-infective (including anti-microbial agents);
an anti-inflammatory;
an anti-parasitic agent;
an anti-Parkinsonism drug:
an anti-pyretic (including analgesic anthpyretics);
an anti-tussivelcough suppressant;
a bronchodilator,
an appetite stimulant;
a cardiovascular drug (including anti-hypertensives);
a decongestant;
a drug for treating gastric disorders;
a drug for renal failure;
a drug which selectively modifies CNS function;
an expectorant;
a general non-selective CNS depressant-,
a general non-selective CNS stimulant;
an H₂-antagonist;
a narcotic analgesic;
a non-steroidal anti-Inflammatory drug;
oral insulin;
a PDE6 Inhibitor,
a proton pump Inhibitor,
a psychopharmacological drug; or
a wound-healing drug.

14. A dosage form according to Claim 13, wherein the pharmaceutically active agent le Ibuprofan, ivermectin, or any form of eletriptan.

15. A dosage form according to Claim 14, wherein the pharmaceutically active agent is eletriptan hydrobromide (Relpax™) or eletriptan hemisulphate.

16. A dosage form according to any of Claims 7 to 15, wherein the pharmaceutically active agent is present at a concentration of from 0.1 to 75% w/w.

17. A dosage form according to any of Claims 7 to 16, wherein the pharmaceutically active agent is an oral healthcare product.

18. A dosage form according to Claim 17, wherein the oral healthcare product is one or more of a deodorising agent, an anti-microbial agent, or a salivary stimulant.

19. A dosage form according to Claim 17 or 18, wherein the oral, healthcare product Is present at a concentration of from 0.1 to 15% w/w.

20. A dosage form according to any of Claims 7 to 19, which dosage form is in the form of a film.

21. A dosage term according to any of Claims 7 to 20, which dosage form is orally consumable.

22. A dosage form according to any of Claims 7 to 21, which dosage term is suitable for human or veterinary use.

## Patentansprüche

1. Verfahren zur Herstellung einer Dosierungsform, die eine Lösung mit niedriger Viskosität ergibt, wenn sie in den Mund des Konsumenten gebracht wird, wobei das Verfahren die folgenden Schritte umfasst:
(a) Herstellen einer hydratisierten Polymerzusammensetzung, die bis zu 70 Gew.-% Pullulan, bis zu 6,0 Gew.-% Natriumalginat und ein pharmazeutisch aktives Mittel oder mehrere pharmazeutisch aktive Mittel umfasst, wobei die Zusammensetzung außerdem eines oder beide der Enzyme Pullulanase oder Alginatlyase umfasst;
(b) Gießen der Zusammensetzung in die Gestalt einer Dosierungsform und
(c) Trocknen der Dosierungsform unter Bereitstellung einer Form, die sich im Mund des Konsumenten schnell löst und dispergiert.

2. Verfahren zur Herstellung einer Dosierungsform, die eine Lösung mit niedriger Viskosität ergibt, wenn sie in den Mund des Konsumenten gebracht wird, wobei das Verfahren die folgenden Schritte umfasst:
(a) Herstellen einer hydratisierten Polymerzusammensetzung, die bis zu 70 Gew.-% Pullulan, bis zu 5,0 Gew.-% Natriumalginat und ein pharmazeutisch aktives Mittel oder mehrere pharmazeutisch aktive Mittel umfasst;
(b) Gießen der Zusammensetzung in die Gestalt einer Dosierungsform;
(c) Trocknen der Dosierungsform und
(d) Bestrahlen der Dosierungsform mit gamma-Strahlung, um eine Form bereitzustellen, die sich im Mund des Konsumenten schnell löst und dispergiert.

3. Verfahren nach Anspruch 2, wobei die gamma-Bestrahlung in einer Menge von 25 kGy oder 40 kGy erfolgt.

4. Verfahren nach einem der Ansprüche 1 bis 3, wobei die bei Auflösung der resultierenden Dosierungsform im Mund des Konsumenten gebildete Lösung eine Viskosität hat, die weniger als 80% derjenigen der in Schritt (a) gebildeten Zusammensetzung ist.

5. Verfahren nach einem der Ansprüche 1 bis 4, wobei Schritt (c) in einem Umluftofen bei einer Temperatur von 50°C bis 80°C für einen Zeitraum von 15 bis 90 Minuten durchgeführt wird.

6. Verfahren nach einem der Ansprüche 1 bis 4, wobei Schritt (c) in einer Beschichtungsmaschine bei einer Temperatur von 20°C bis 150°C durchgeführt wird.

7. Dosierungsform, die nach einem Verfahren, das in einem der Ansprüche 1 bis 6 beschrieben ist, erhältlich ist.

8. Dosierungsform nach Anspruch 7, in der Pullulan in einer Menge von 5 bis 45 Gew.-% vorliegt.

9. Dosierungsform nach Anspruch 8, in der Pullulan in einer Menge von 15 bis 25 Gew.-% vorliegt.

10. Dosierungsform nach Anspruch 9, in der Pullulan in einer Menge von 20 Gew.-% vorliegt.

11. Dosierungsform nach Anspruch 7, in der Natriumalginat in einer Menge von 0,1 bis 2,5 Gew.-% vorliegt.

12. Dosierungsform nach Anspruch 11, in der Natriumalginat in einer Menge von 0,5 Gew.-% vorliegt.

13. Dosierungsform nach einem der Ansprüche 7 bis 12, in der das pharmazeutisch aktive Mittel ist:
ein Anticholesterolämikum;
ein Antidiarrhoikum;
ein Antiemetikum;
ein Antimykotikum;
ein Antihistaminikum;
ein Antiinfektivum (einschließlich antimikrobieller Mittel);
ein entzündungshemmendes Mittel;
ein Antiparasitikum;
ein Antiparkinsonikum;
ein Antipyretikum (einschließlich analgetischer Antipyretika);
ein Antitussivum/Mittel zur Unterdrückung von Husten;
ein Bronchodilatator;
ein appetitanregendes Mittel;
ein Herz-Kreislauf-Arzneimittel (einschließlich Antihypertensiva);
ein Entstauungsmittel;
ein Arzneimittel zur Behandlung von Magenbeschwerden;
ein Arzneimittel gegen Nierenversagen;
ein Arzneimittel, das die ZNS-Funktion selektiv modifiziert;
ein Expektorans;
ein allgemeines nicht-selektives ZNS-Sedativum;
ein allgemeines nicht-selektives ZNS-Stimulans;
ein H₂-Antagonist;
ein narkotisches Analgetikum;
ein nicht-steroidales Antirheumatikum;
orales Insulin;
ein Protonenpumpe-Inhibitor;
ein psychopharmakologisches Arzneimittel oder
ein Arzneimittel zur Wundheilung.

14. Dosierungsform nach Anspruch 13, worin das pharmazeutisch aktive Mittel Ibuprofen, Ivermectin oder eine Form von Eletriptan ist.

15. Dosierungsform nach Anspruch 14, worin das pharmazeutisch aktive Mittel Eletriptan-Hydrobromid (Relpax™) oder Eletriptan-Hemisulfat ist.

16. Dosierungsform nach einem der Ansprüche 7 bis 15, worin das pharmazeutisch aktive Mittel in einer Konzentration von 0,1 bis 75 Gew.-% vorliegt.

17. Dosierungsform nach einem der Ansprüche 7 bis 16, worin das pharmazeutisch aktive Mittel ein orales Gesundheitsfürsorgeprodukt bzw. ein orales Health-Care-Produkt ist.

18. Dosierungsform nach Anspruch 17, worin das orale Gesundheitsfürsorgeprodukt eines oder mehrere aus einem desodorierenden Mittel, einem antimikrobiellen Mittel oder einem Speichelstimulans ist.

19. Dosierungsform nach Anspruch 17 oder 18, worin das orale Gesundheitsfürsorgeprodukt in einer Konzentration von 0,1 bis 15 Gew.-% vorliegt.

20. Dosierungsform nach einem der Ansprüche 7 bis 19, wobei die Dosierungsform in der Form eines Films ist.

21. Dosierungsform nach einem der Ansprüche 7 bis 20, wobei die Dosierungsform oral konsumierbar ist.

22. Dosierungsform nach einem der Ansprüche 7 bis 21, wobei die Dosierungsform zur humanen oder veterinären Verwendung geeignet ist.

## Revendications

1. Procédé de préparation d'une forme posologique qui libère une solution de faible viscosité lorsqu'elle est placée dans la bouche d'un consommateur, ledit procédé comprenant les étapes suivantes :
(a) préparation d'une composition polymère hydratée comprenant jusqu'à 70 % en poids de pullulan, jusqu'à 5,0 % en poids d'alginate de sodium et un ou plusieurs agent(s) pharmaceutiquement actif(s), ladite composition comprenant en outre l'une des enzymes pullulanase ou alginate lyase, ou les deux ;
(b) transformation de ladite composition en une forme posologique ; et
(c) séchage de ladite forme posologique pour obtenir une forme qui se dissout et se disperse rapidement dans la bouche du consommateur.

2. Procédé de préparation d'une forme posologique qui libère une solution de faible viscosité lorsqu'elle est placée dans la bouche d'un consommateur, ledit procédé comprenant les étapes suivantes :
(a) préparation d'une composition polymère hydratée comprenant jusqu'à 70 % en poids de pullulan, jusqu'à 5,0 % en poids d'alginate de sodium et un ou plusieurs agent(s) pharmaceutiquement actif(s) ;
(b) coulage de ladite composition à la configuration d'une forme posologique ;
(c) séchage de ladite forme posologique ; et
(d) irradiation de ladite forme posologique avec une radiation gamma pour obtenir une forme qui se dissout et se disperse rapidement dans la bouche du consommateur.

3. Procédé selon la revendication 2, dans lequel ladite irradiation gamma est de 25 kGy ou de 40 kGy.

4. Procédé selon l'une quelconque des revendications 1 à 3, dans lequel la solution formée par dissolution de la forme posologique résultante dans la bouche du consommateur a une viscosité qui est inférieure à 80 % de celle de la composition formée à l'étape (a).

5. Procédé selon l'une quelconque des revendications 1 à 4, dans lequel l'étape (c) est mise en oeuvre dans un four ventilé à une température allant de 50°C à 80°C, pendant une période allant de 15 à 90 minutes.

6. Procédé selon l'une quelconque des revendications 1 à 4, dans lequel l'étape (c) est mise en oeuvre dans une machine d'enduction à une température allant de 20°C à 150°C.

7. Forme posologique pouvant être obtenue selon un procédé décrit dans l'une quelconque des revendications 1 à 6.

8. Forme posologique selon la revendication 7, dans laquelle le pullulan est présent en une quantité allant de 5 à 45 % en poids.

9. Forme posologique selon la revendication 8, dans laquelle le pullulan est présent en une quantité allant de 15 à 25 % en poids.

10. Forme posologique selon la revendication 9, dans laquelle le pullulan est présent en une quantité de 20 % en poids.

11. Forme posologique selon la revendication 7, dans laquelle l'alginate de sodium est présent en une quantité allant de 0,1 à 2,5 % en poids.

12. Forme posologique selon la revendication 11, dans laquelle l'alginate de sodium est présent en une quantité de 0,5 % en poids.

13. Forme posologique selon l'une quelconque des revendications 7 à 12, dans laquelle l'agent pharmaceutiquement actif est :
· un anticholestérolémiant ;
· un antidiarrhéique ;
· un antiémétique ;
· un antifongique;
· un antihistaminique;
· un anti-infectieux (y compris les agents antimicrobiens) ;
· un anti-inflammatoire;
· un agent antiparasitaire;
· un médicament antiparkinsonien;
· un antipyrétique (y compris les analgésiques antipyrétiques) ;
· un antitussif/médicament contre la toux ;
· un bronchodilatateur ;
· un stimulant de l'appétit ;
· un médicament cardio-vasculaire (y compris les antihypertenseurs) ;
· un décongestionnant ;
un médicament pour le traitement des troubles gastriques ;
· un médicament contre l'insuffisance rénale ;
un médicament qui modifie sélectivement le fonctionnement du SNC ;
· un expectorant ;
· un dépresseur général, non-sélectif, du SNC ;
· un stimulant général, non-sélectif, du SNC ;
· un antagoniste H2 ;
· un analgésique narcotique;
· un médicament anti-inflammatoire non-stéroïdien ;
· de l'insuline orale ;
· un inhibiteur des PDE5
· un inhibiteur de la pompe à protons ;
· un médicament psychopharmacologique ; ou
· un médicament cicatrisant.

14. Forme posologique selon la revendication 13, dans laquelle l'agent pharmaceutiquement actif est l'ibuprofène, l'ivermectine, ou l'élétriptan sous toutes ses formes.

15. Forme posologique selon la revendication 14, dans laquelle l'agent pharmaceutiquement actif est le bromhydrate d'élétriptan (Relpax™) ou l'hémisulfate d'élétriptan.

16. Forme posologique selon l'une quelconque des revendications 7 à 15, dans laquelle l'agent pharmaceutiquement actif est présent à une concentration allant de 0,1 à 75 % poids/poids.

17. Forme posologique selon l'une quelconque des revendications 7 à 16, dans laquelle l'agent pharmaceutiquement actif est un produit d'hygiène bucco-dentaire.

18. Forme posologique selon la revendication 17, dans laquelle le produit d'hygiène bucco-dentaire est l'un ou plusieurs des agents suivants : un agent déodorant, un agent antimicrobien ou un stimulant salivaire.

19. Forme posologique selon la revendication 17 ou 18, dans laquelle le produit d'hygiène bucco-dentaire est présent à une concentration allant de 0,1 à 15 % poids/poids.

20. Forme posologique selon l'une quelconque des revendications 7 à 19, ladite forme posologique étant sous la forme d'un film.

21. Forme posologique selon l'une quelconque des revendications 7 à 20, ladite forme posologique étant consommable par voie orale.

22. Forme posologique selon l'une quelconque des revendications 7 à 21, ladite forme posologique convenant à l'utilisation humaine ou vétérinaire.
